# EUROPÄISCHE PATENTSCHRIFT

(11) **EP 1 107 749 B1**
(45) Veröffentlichungstag und Bekanntmachung des Hinweises auf die Patenterteilung: **28.05.2003**
(21) Anmeldenummer: 99944453.2
(22) Anmeldetag: 20.08.1999
(51) Int. Cl.: A61K 31/225, A61P 37/06, A61K 38/13

(54) **FUMARSÄUREDERIVATE ZUR BEHANDLUNG DER TRANSPLANTATABSTOSSUNG**
FUMARIC ACID DERIVATIVES FOR THE TREATMENT OF TRANSPLANT REJECTION
DERIVES D'ACIDE FUMARIQUE POUR LE TRAITEMENT DU REJET DE GREFFE

(30) Priorität: 31.08.1998 DE 19839566
(43) Veröffentlichungstag der Anmeldung: 20.06.2001
(73) Patentinhaber: Fumapharm AG, 5630 Muri (CH)
(72) Erfinder: JOSHI, Rajendra, Kumar, CH-8048 Zürich (CH); STREBEL, Hans-Peter, CH-5630 Muri (CH)
(74) Vertreter: Schwabe - Sandmair - Marx
(86) Internationale Anmeldenummer: EP9906110
(87) Internationale Veröffentlichungsnummer: WO00012072

(56) Entgegenhaltungen:
- EP-A- 0 312 697
- WO-A-96/27369
- WO-A-98/04290
- WO-A-98/27970
- WO-A-98/52549
- NATHENS, AVERY B. ET AL: "The glutathione depleting agent diethylmaleate prolongs renal allograft survival" J. SURG. RES. (1998), 77(1), 75-79 , XP000884311
- SCHWINGHAMMER T L ET AL: "PHARMACOLOGIC PROPHYLAXIS OF ACUTE GRAFT-VERSUS-HOST DISEASE AFTER ALLOGENEIC MARROW TRANSPLANTATION" CLINICAL PHARMACY,US,AMERICAN SOCIETY OF HOSPITAL PHARMACISTS, Bd. 12, Nr. 10, 1. Oktober 1993 (1993-10-01), Seiten 736-761, XP000564975 ISSN: 0278-2677
- GASSER M ET AL: "Host vs graft and graft vs host reactions after allogeneic heterotopic small bowel transplantation in the rat." TRANSPLANTATION PROCEEDINGS, (1992 JUN) 24 (3) 1128-9. , XP000884321

## Beschreibung

Die Erfindung betrifft die Verwendung bestimmter Fumarsäuremonoalkylester entweder in Form ihrer Salze oder als freie Säure, allein oder in Kombination mit einem Dialkylfumarat zur Herstellung von pharmazeutischen Zubereitungen zur Anwendung in der Transplantationsmedizin. Insbesondere betrifft sie die Verwendung zur Herstellung dieser Fumarsäurealkylester enthaltenden pharmazeutischen Zubereitungen zur Behandlung, Linderung oder Unterdrückung von Abstoßungsreaktionen des Empfängers gegen das Transplantat d.h. Host-versus-Graft-Reaktionen.

Transplantationen sind Gewebe- bzw. Organverpflanzungen, d.h. Übertragungen von Geweben wie Hornhaut, Haut, Knochen (Knochenspänen), Gefäßen oder Faszien, von Organen, wie Niere, Herz, Leber, Lunge, Bauchspeicheldrüse oder Darm oder auch einzelner Zellen wie Inselzellen, Alphazellen und Leberzellen, worunter der Niere als transplantiertem Organ die wichtigste Bedeutung zukommt.

Nach dem Verwandschaftsgrad von Spender und Empfänger unterscheidet man die Autotransplantation (Übertragung auf einen anderen Körperteil des gleichen Individuums), die Isotransplantation (Übertragung auf ein anderes, genetisch gleiches Individuum) und die allogene Transplantation (Übertragung auf ein anderes Individuum der gleichen Art). Nach Herkunfts- und Transplantationsort unterscheidet man weiterhin die homotope Transplantation (Übertragung auf den gleichen Ort) und die heterotope Transplantation (Übertragung auf einen anderen Ort). Den genannten Transplantationen kommt in der modernen Medizin eine bedeutende Rolle zu.

Probleme bereitet in der Transplantationsmedizin vor allem die Transplantatabstoßung (Englisch: graft-rejection) nach der Transplantation des Gewebes, Organs oder der Zellen durch Immunabwehrreaktionen des Empfängers. Die Transplantatabstoßung wird auch Host-versus-Graft-Reaktion genannt. Die immunologischen Abwehrreaktion des Organismus gegen das Fremdprotein führt oft zur Abstoßung oder Auflösung der Transplantate. Durch Einsatz moderner Immunsupressiva, für die als wichtigste Vertreter die Cyclosporine, insbesondere Cyclosporin A zu nennen sind, konnten die Transplantationsresultate in den letzten Jahren erheblich verbessert werden. Die 1-Jahres-Überlebensrate beträgt zur Zeit für Lebertransplantationen etwa 60 %, für Herztransplantationen ca. 80 % und für Nierentransplantationen über 90 %.

Bei den Host-versus-Graft-Reaktionen lassen sich verschiedene Stufen unterscheiden. Und zwar erfolgt die Reaktion je nach Grad der Verschiedenheit zwischen Empfänger und Spender unterschiedlich schnell, so daß man von akuter, subakuter oder chronischer Reaktion spricht. Die akut verlaufende Abstoßungsreaktion geht mit dem irreversiblen, medikamentös nicht beeinflußbaren Verlust (Nekrotisierung) des Transplantats in Folge Arteriitis bzw. Arteriolitis innerhalb von 48 Stunden einher. Die subakute Abstoßungsreaktion zeigt sich als Abstoßungskrise ab dem 12. Tag bis zum 4. Monat mit reversiblen Funktionsstörungen in Folge Transplantatvaskulopathie. Schließlich wird als chronische Abstoßungsreaktion der über Wochen bis Jahre fortschreitende, medikamentös kaum beeinflußbare Funktionverlust des Transplantats in Folge vaskulärer Veränderungen bspw. obliterierender Arteriopathie bezeichnet.

Zur Vermeidung dieser Abstoßungsreaktionen d.h. der Host-versus-Graft-Reaktion bedient sich die Transplantationsmedizin im wesentlichen der Immunsuppression, also der Abschwächung der normalen Immunantwort. Hierzu setzt man häufig Anti-Lymphozytenseren in Kombination mit Corticosterioden und sogenannten Antimetaboliten bspw. Purinanaloga wie 6-Mercaptopurin und Thioguanin ein, die die Nukleinsäure- und Proteinsynthese stören und damit die Zellteilung und Proliferation verhindern. Dies führt zur Unterdrückung der Antikörperproduktion und der zellulären Immunantwort. Bei den zur Therapie verwendeten Immunsupressiva handelt es sich um Substanzen, die die Immunreaktion im Körper entweder spezifisch oder unspezifisch unterdrücken bzw. abschwächen. Unspezifische Immunsuppresiva sind Zytostatika wie bspw. Alkylantien oder Antimetabolite. Daneben kennt man Wirkstoffe, die zumindest teilweise eine spezifische Immunsuppression bewirken wie Corticosteriode, Antiseren, Antikörper FK-506, Tacrolimus, Mycophenolatmofetil und hauptsächlich die Cyclosporine, wie Cyclosporin A.

Die Gefahr bei der Anwendung von Immunsuppressiva liegt in der Schwächung der körpereigenen Abwehr gegen Infektionskrankheiten und dem erhöhten Risikos maligner Erkrankungen. Die Aufgabe der Erfindung besteht daher darin, eine pharmazeutische Zubereitung zur Verwendung in der Transplantationsmedizin bereitzustellen, welche zur Behandlung, insbesondere Unterdrückung, Abschwächung und/oder Linderung der Hostversus-Graft-Reaktionen verwendet werden kann, diesen Nachteil jedoch nicht aufweist.

Die Lösung der erfindungsgemäßen Aufgabe liegt in der Verwendung bestimmter Fumarsäuremonoalkylester als Salze mit ein- oder zweiwertigen Kationen oder in Form der freien Säure, allein oder in Kombination mit einem Dialkylfumarat zur Herstellung von pharmazeutischen Zubereitungen zur Verwendung in der Transplantationsmedizin. Die erfindungsgemäßen Gegenstände sind in den Ansprüchen im einzelnen gekennzeichnet. Die erfindungsgemäßen Zubereitungen enthalten keine freie Fumarsäure per se.

Bekannt ist, daß pharmazeutische Zubereitungen, die nach Verabreichung bei ihrem biologischen Abbau in den Zitronensäurezyklus einmünden oder diesem angehören, zumeist in hoher Dosierung immer mehr an therapeutischem Wert gewinnen, da man mit ihrer Hilfe kryptogenetisch bedingte Krankheiten zu lindern oder zu heilen vermag.

So hemmt Fumarsäure das Wachstum des Ehrlich-Ascites-Tumors bei Mäusen, vermindert die toxischen Effekte von Mitoycin C und Aflatoxin (K. Kuroda, M. Akao, Biochem. Pharmacol. 29, 2839-2844 (1980) / Gann. 72, 777-782 (1981) / Cancer Res. 36, 1900-1903 (1976)) und besitzt eine antipsoriatische sowie antimikrobielle Wirkung [C. N. Huhtsnen, J. Food Sci. 48, 1574 (1983) / M. N. Islam, U.S.-Patent 4 346 118 vom 24. August 1982 / C.A. 97, 161317b (1982)].

Hohe Verabreichungsdosen von Fumarsäure oder ihren bisher bekannten Derivaten wie Dihydroxylfumarsäure, Fumaramid und Fumaronitril besitzen bei parenteraler, dermaler, insbesondere aber peroraler Verabreichung eine derart unzumutbare Nebenwirkungsrate und hohe Toxität (P. Holland, R.G. White, Brit. Dernatol, 85, 259-263 (1971) / M. Hagedorn, K.W. Kalkoff, G. Kiefer, D. Baron, J. Hug, J. Petres, Arch. Der. Res. 254, 67-73 (1975)), daß bisher meist von einer solchen Therapie abgesehen werden mußte.

In der europäischen Patentanmeldung 18 87 49 sind bereits Fumarsäurederivate und sie enthaltende pharmazeutische Zubereitungen zur Behandlung der Psoriasis beschrieben. Aus der DE-A-25 30 372 sind pharmazeutische Zubereitungen zur Behandlung der Psoriasis bekannt, die eine Mischung aus Fumarsäure und weiteren Fumarsäurederivaten enthalten. Der Anteil an freier Fumarsäure ist bei diesen Arzneimitteln obligatorisch.

Die DE-A-26 21 214 beschreibt Arzneimittel zur Behandlung der Psoriasis, die den Fumarsäuremonoethylester und dessen Mineralsalze als Wirkstoff enthalten. Aus der Publikation "Hautarzt (1987) 279-285" ist die Verwendung von Fumarsäuremonoethylestersalzen bekannt. Aus dem Patent EP 0 312 697 B1 sind pharmazeutische Zubereitungen zur Behandlung der Psoriasis, psoriatischer Arthritis, Neurodermitis und Enteritis regionalis Crohn bekannt, die eine Mischung aus Fumarsäuremonoalkylestersalzen und einem Fumarsäurediester enthalten.

Es wurde nun überraschend gefunden, daß Fumarsäure-C₁₋₅-monoalkylerester in Form ihrer Salze mit ein oder zweiwertigen Kationen, vorzugsweise in Form der Calcium-, Magnesium-, Zink- oder Eisensalze bzw. der Lithium-, Natrium- oder Kaliumsalze, oder als freie Säure, allein oder in Kombination mit einem Di-C₁₋₅-alkylfumarat vorteilhaft zur Herstellung einer pharmazeutischen Zubereitung zur Verwendung in der Transplantationsmedizin verwendet werden können. Die diese Fumarsäure -C₁₋₅-monoalkylester enthaltenden Zubereitungen gestatten überraschenderweise eine positive Modulation des Immunsystems in der Host-versus-Graft-Reaktion.

Vorzugsweise verwendet man erfindungsgemäß pharmazeutische Zubereitungen, die eine oder mehrere Verbindungen aus der Gruppe der Calcium-, Magnesium-, Zink- und Eisensalze bzw. Lithium-, Natrium-, oder Kaliumsalze von Fumarsäuremonoalkylestern der allgemeinen Formel 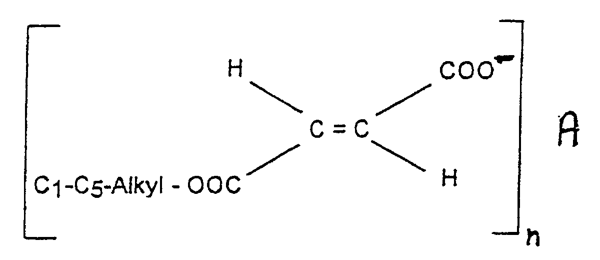 gegebenenfalls im Gemisch mit Dialkylfumarat der Formel 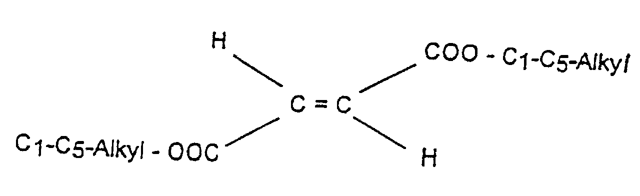 gegebenenfalls neben üblichen pharmazeutischen Hilfs- und Trägerstoffen enthalten,
wobei A ein zweiwertiges Kation der Reihe Ca, Mg, Zn oder Fe bzw. ein einwertiges Kation aus der Reihe Li, Na, oder K und n 1 oder 2 je nach Art des Kations bedeuten.

Ebenso vorteilhaft können pharmazeutische Zubereitungen verwendet werden, die eine oder mehrere Alkylhydrogenfumarate der allgemeinen Formel 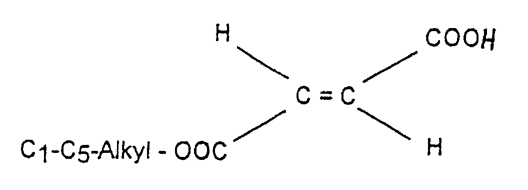 gegebenenfalls im Gemisch mit Dialkylfumarat der Formel 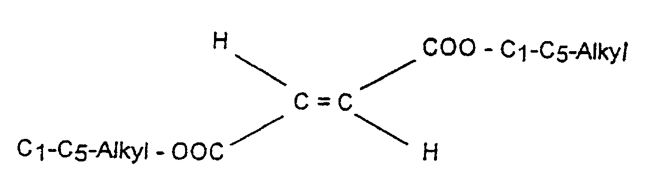 neben gegebenenfalls üblichen pharmazeutischen Hilfs- und Trägerstoffen enthalten.

Bervorzugt ist eine Verwendung, bei der die Wirkstoffe oral in Form von Tabletten, von Mikrotabletten, Pellets oder Granulaten in Kapseln oder (Weich- bzw. Hartgelatine-) Kapseln verabreicht werden.

Bevorzugte Zubereitungen gemäß der Erfindung enthalten das Calciumsalz des Fumarsäuremonomethylesters oder -monoethylesters oder das Calciumsalz des Fumarsäuremonomethylesters bzw. -monoethylesters im Gemisch mit Dimethylfumarat.

Zur Verabreichung sind besonders Zubereitungen geeignet, die das Calciumsalz des Fumarsäuremonoalkylesters bzw. den Fumarsäurealkylester in Form der freien Säure in einer Menge von 10 bis 300 mg enthalten, wobei das Gesamtgewicht der Wirkstoffe 10 bis 300 beträgt.

Weitere bevorzugte orale Verabreichungsformen enthalten 10 bis 290 Gew.-Teile des Calciumsalzes des Fumarsäuremonoalkylesters und 290 bis 10 Gew.-Teile Dimethylfumarat sowie 1 bis 50 Gew.-Teile des Zinksalzes des Fumarsäuremonoalkylesters oder 1 bis 250 Gew.-Teile Dimethylfumarat, 1 bis 50 Gew.-Teile des Magnesiumsalzes des Fumarsäuremonoalkylesters bspw, des Monomethylesters, wobei jeweils das Gesamtgewicht der Wirkstoffe 30 bis 300 mg beträgt.

Bevorzugte Zubereitungen gemäß der Erfindung enthalten auch das Methylhydrogenfumarat in einer Menge von 10 bis 300 mg.

Außerdem möglich ist eine Verwendung, bei der die Arzneimittel in Form von Präparaten für die kutane und transdermale Verabreichung, Präparaten für die parenterale Verabreichung und Präparaten für die rektale Verabreichung eingesetzt werden.

Die in den erfindungsgemäßen Zubereitungen enthaltenen Fumarsäurederivate werden beispielsweise dadurch erhalten, daß man eine Verbindung der folgenden Formel 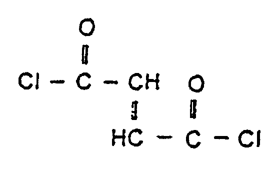
a) mit 2 Mol Alkylalkohol (ROH) in bekannter Weise zum Diester kondensiert und anschließend kontrolliert zum Monoester hydrolysiert, oder
b) mit 1 Mol eines entsprechenden Alkylalkohols in üblicher Weise kondensiert und das enthaltene Monosäurechlorid zur Säure hydrolisiert, oder
c) die Fumarsäure direkt mit 2 Mol Alkylalkohol (ROH) in bekannter Weise zu dem jeweiligen Diester kondensiert und anschließend kontrolliert zum Monoester hydrolysiert, oder
d) Maleinsäure oder Maleinsäureanhydrid direkt mit 1 - 2 Mol des entsprechenden Alkylalkohols (ROH) in bekannter Weise zu einem Mono- oder Diester kondensiert und anschließend katalytisch zum entsprechenden Fumarsäurederivat isomerisiert.

Die Salze der Fumarsäuremonoalkylester können dadurch erhalten werden, daß man eine Verbindung der allgemeinen Formel 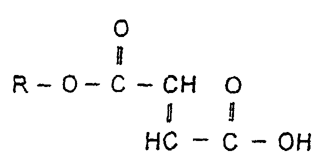 in der R eine C₁-C₅-Alkylgruppe bedeutet, mit äquivalenten Molmengen Na-, K-, Fe-, Ca-, Mg- oder Zn-Hydroxid oder -Oxid in Toluol zur Umsetzung bringt und das während der Reaktion gebildete Wasser entfernt.

Besonders bevorzugt werden insbesondere Arzneimittel mit den folgenden Wirkstoffen in den bezeichneten Dosierungen und Mengenverhältnissen eingesetzt:
1) pharmazeutische Zubereitungsform zur oralen Verabreichung in Form von Tabletten, Mikrotabletten oder Pellets in Kapseln oder Kapseln, dadurch gekennzeichnet, daß sie das Calciumsalz des Fumarsäuremonomethylesters in einer Menge von 10 bis 300 mg enthält, wobei das Gesamtgewicht der Wirkstoffe 10 bis 300 mg beträgt oder als
2) pharmazeutische Zubereitungsform zur oralen Verabreichung in Form von Tabletten, Mikrotabletten oder Pellets in Kapseln oder Kapseln, dadurch gekennzeichnet, daß sie 10 bis 290 Gewichtsteile des Calciumsalzes des Fumarsäuremonomethylesters und 290 bis 10 Gewichtsteile Dimethylfumarat enthalten, wobei das Gesamtgewicht der Wirkstoffe 20 bis 300 mg beträgt,
3) pharmazeutische Zubereitungsform zur oralen Verabreichung in Form von Tabletten, Mikrotabletten oder Pellets in Kapseln oder Kapseln, dadurch gekennzeichnet, daß sie jeweils 10 bis 250 Gewichtsanteile des Calciumsalzes des Fumarsäuremonomethylesters, 1 bis 50 Gewichtsteile Dimethylfumarat und 1 bis 50 Gewichtsteile des Zinksalzes des Fumarsäuremonomethylesters enthalten, wobei das Gesamtgewicht der Wirkstoffe 20 bis 300 mg beträgt, oder
4) pharmazeutische Zubeitungsform zur oralen Verabreichung in Form von Tabletten oder Kapseln, dadurch gekennzeichnet, daß sie 10 bis 250 Gewichtsteile des Calciumsalzes des Fumarsäuremonomethylesters, 250 bis 10 Gewichtsteile Dimethylfumarat, 1 bis 50 Gewichtsteile des Magnesiumsalzes des Fumarsäuremonomethylesters und 1 bis 50 Gewichtsteile des Zinksalzes des Fumarsäuremonomethylesters enthalten, wobei das Gesamtgewicht der Wirkstoffe 30 bis 300 mg beträgt.

Bevorzugt sind weiterhin folgende Arzneiformen, Dosierungen und Mengenverhältnisse:
5) pharmazeutische Zubereitung zur oralen Verabreichung, dadurch gekennzeichnet, daß sie eine oder mehrere Verbindungen aus der Gruppe der freien Säuren von Fumarsäuremonoalkylestern der allgemeinen Formel 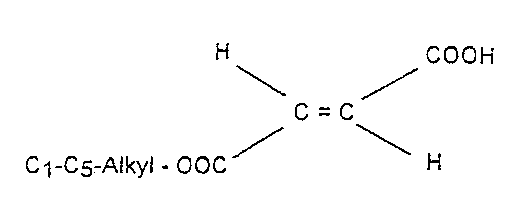 gegebenenfalls mit einem Gemisch mit Dialkylfumarat der Formel 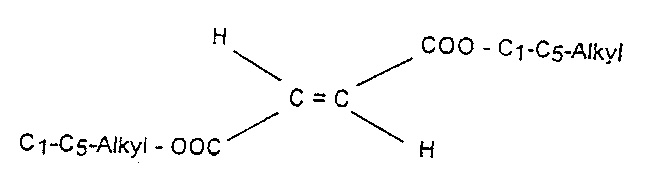 und Trägerstoffe enthält. wobei diese Zubereitungen Fumarsäure in freier Form nicht enthalten,
6) pharmazeutische Zubereitungsform zur oralen Verabreichung in Form von Tabletten, Mikrotabletten oder Pellets in Kapseln oder Kapseln, dadurch gekennzeichnet, daß sie Alkylhydrogenfumarat, vorzugsweise Methylhydrogenfumarat, in einer Menge von 10 bis 300 mg enthalten, wobei das Gesamtgewicht der Wirkstoffe 10 bis 300 mg beträgt oder
7) pharmazeutische Zubereitungsform zur oralen Verabreichung in Form von Tabletten, Mikrotabletten oder Pellets in Kapseln oder Kapseln, dadurch gekennzeichnet, daß sie 10 bis 290 Gewichtsteile Alkylhydrogenfumarat, vorzugsweise Methylhydrogenfumarat, und 290 bis 10 Gewichtsteile Dialkylfumarat enthalten, wobei das Gesamtgewicht der Wirkstoffe 20 bis 300 mg beträgt.

Orale Zubereitungen können mit einem magensaftresistenten Überzug versehen sein. Die Zubereitungen können in Form von Mikrotabletten, Pellets oder Granulaten vorliegen, die gegebenenfalls in Kapseln enthalten sind. Kapseln können Weich- oder HartgelatineKapseln sein.

Nach einer vorzugsweise Ausgestaltung liegt die Größe bzw. der mittlere Durchmesser der Pellets oder Mikrotabletten im Bereich von 300 bis 2000 µm, insbesondere im Bereich von 500 µm oder 1000 µm.

Die Therapie kann mit Fumarsäuremonoalkylestern bzw. deren Salzen auch in Kombination mit einem oder mehreren Präparaten der bei Organtransplantationen üblicherweise eingesetzten Tripledrugtherapie oder auch mit Cyclosporin A insbesondere zur Behandlung, Linderung und Unterdrückung von Host-versus-Graft-Reaktionen erfolgen.

Hierzu können die verabreichten Präparate eine Kombination der Wirkstoffe in den bekannten Dosierungen bzw. Mengen enthalten. Ebenso kann die Kombinationstherapie in der parallen Verabreichung separater Präparate auf gleichem oder unterschiedlichen Applikationsweg bestehen. Gegebenenfalls kann die Dosis des neben der erfindungsgemäß verabreichten Fumarsäurederivatdosis enthaltenen Wirkstoffs in vorteilhafter Weise gesenkt werden.

Eine weitere Ausgestaltung der Verwendung kann darin bestehen, die Arzneitherapie mit Immunsuppressiva wie Cyclosporin sequentiell mit einer Applikation der oben bezeichneten Fumarsäurederivate zu alternieren. Das heißt, daß nach ein- bis mehrwöchiger Cyclosporin-Therapie eine ein- bis mehrwöchige Applikation von Fumarsäurederivaten gemäß vorstehender Bedeutung erfolgen kann. Dadurch läßt sich die Dosierung von Cyclosporin A senken wodurch eine bedeutend verringtere Nebenwirkungsrate bei der Langzeittherapie erreicht werden kann.

Im folgenden werden zur Erläuterung der erfindungsgemäßen Verwendung verschiedene Beispiele für die Herstellung bevorzugter Arzneimittel gegeben:

### Herstellungsbeispiele

### Beispiel 1

### Herstellung von Filmtabletten mit magensaftresistentem Überzug enthaltend 100,0 mg Monoethylfumarat-Ca-Salz, entsprechend 71 mg Fumarsäure

10,000 kg Monoethylfumarat-Ca-Salz werden zerkleinert, intensiv gemischt und unter entsprechenden Vorsichtsmaßnahmen (Atemmaske, Handschuhe, Schutzanzug etc.) mittels eines Siebes 800 homogenisiert. Anschließend wird ein Hilfsstoffgemisch folgender Zusammensetzung hergestellt: 21,000 kg Stärkederivat (STA-RX 1500®), 2,000 kg mikrokristalline Cellulose (Avicel PH 101®), 0,600 kg Polyvinylpyrrolidon (PVP, Kollidon®25), 4,000 kg Primogel®, 0,300 kg kollodiale Kieselsäure (Aerosil®).

Das gesamte Pulvergemisch wird mit dem Wirkstoff versetzt, gemischt, mittels eines Siebes 200 homogenisiert und mit einer 2 %-igen wäßrigen Lösung von Polyvinylpyrrolidon (PVP, Kollidon®25) auf übliche Weise zu einem Bindemittelgranulat verarbeitet und in trockenem Zustand mit der äußeren Phase gemischt. Diese besteht aus 2,000 kg eines sogenannten FST-Komplexes, enthaltend 80 % Talk, 10 % Kieselsäure und 10 % Magnesiumstearat.

Es wird anschließend auf übliche Weise zu gewölbten Tabletten von 400 mg Gewicht und 10,0 mm Durchmesser gepreßt. Anstelle dieser klassischen Tablettiermethoden können auch andere Methoden zur Herstellung von Tabletten angewendet werden, wie Direkttablettierung sowie feste Dispersionen nach der Schmelzmethode und der Sprühtrocknungsmethode.

### Magensaftresistenz

Es wird eine Lösung von 2,250 kg Hydroxypropylmethylcellulosephthalat (HPMCP, Pharmacoat HP®50) in einem Lösungsmittelgemisch von 2,50 1 demineralisiertem Wasser, 13,00 1 Aceton Ph.Helv. VII und 13,00 1 Ethanol 94 Gewichtsprozent gelöst und die Lösung mit 0,240 kg Rizinusöl (Ph.Eur. II) versetzt. Die Lösung wird im Dragierkessel auf traditionelle Weise in Portionen auf die Tablettenkerne aufgeleert oder aufgesprüht bzw. in einem Wirbelschichtapparat entsprechender Konstruktion aufgetragen.

Nach entsprechender Trocknung wird anschließend der Filmüberzug angebracht. Dieser setzt sich zusammen aus einer Lösung von Eudragit E 12,5 %® 4,800 kg Talcum Ph. Eur. II 0,340 kg, Titan (VI)-oxid Cronus RN 56® 0,520 kg, Farblack ZLT-2 blau (Siegle) 0,210 kg und Polyethylenglycol 6000 Ph.Helv. VII 0,120 kg in einem Lösungsmittelge-misch von 8,200 kg 2-Propanol Ph.Helv. VII, 0,060 kg Glycerintriacetat (Triacetin®) und 0,200 kg Aqua demineralisata. Nach homogener Verteilung im Dragierkessel oder Wirbelschichtbett wird getrocknet und auf übliche Weise poliert.

### Beispiel 2

### Herstellung von magensaftresistenten Kapseln, enthaltend 86,5 mg Monoethylfumarat-Ca-Salz und 110,0 mg Dimethylfumarat, entsprechend insgesamt 150 mg Fumarsäure

8,650 kg Monoethylfumarat-Ca-Salz und 11,000 kg Dimethylfumarat werden mit einem Gemisch bestehend aus 15,000 kg Stärke, 6,000 kg Lactose Ph. Helv. VII, 2,000 kg mikrokristalliner Cellulose (Avicel®), 1,000 kg Polyvinylpyrrolidon (Kollidon®25) und 4,000 kg Primogel® intensiv gemischt und unter entsprechenden Vorsichtsmaßnahmen (Atemmaske, Handschuhe, Schutzanzug etc.) mittels eines Siebes 800 homogenisiert.

Das gesamte Pulvergemisch wird mit einer 2 %-igen wäßrigen Lösung von Polyvinylpyrrolidon (Kollidon®25) auf übliche Weise zu einem Bindemittelgranulat verarbeitet und in getrocknetem Zustand mit der äußeren Phase gemischt. Diese besteht aus 0,350 kg kolloidaler Kieselsäure (Aerosil®), 0,500 kg Mg-Stearat und 1,500 kg Talkum Ph. Helv. VII. Das homogene Gemisch wird anschließend in entsprechende Kapseln in Portionen von 500,0 mg abgefüllt, welche abschließend auf übliche Weise mit einem magensaftresistenten Überzug, bestehend aus Hydroxypropylethylcellulosephatalat und Rizinusöl als Weichmacher, versehen werden. Die Abfüllung kann ebenfalls anstelle von Hartgelatinekapseln in entsprechende magensaftresistente Kapseln, bestehend aus einem Gemisch von Cellulloseacetatphthalat (CAP) und Hydroxypropyethylcellulosephthalat (HPMCP). erfolgen.

### Beispiel 3

### Herstellung von magensaftresistenten Kapseln, enthaltend 203,0 mg Monoethylfumarat-Ca-Salz, 5,0 mg Monoethylfumarat-Mg-Salz und 3,0 mg Monoethylfumarat-Zn-Salz, entsprechend insgesamt 150 mg Fumarsäure

20.300 kg Monoethylfumarat-Ca-Salz sowie 0,500 kg Monoethylfumarat-Mg-Salz und 0,300 kg Monoethylfumarat-Zn-Salz werden zerkleinert, intensiv gemischt und unter entsprechenden Vorsichtsmaßnahmen (Atemmaske, Handschuhe, Schutzanzug etc.) mittels eines Siebes 800 homogenisiert. Diesem Wirkstoffgemisch wird ein homogenes Pulvergemisch folgender Zusammensetzung untergemischt: sprühgetrocknete Lactose 12,900 kg, kolloidale Kieselsäure 1,000 kg, mikrokristalline Cellulose (Avicel®) 2,000 kg, Magnesiumstearat (Ph. Helv. VII) 1,000 kg und Talk (Ph. Helv. VII) 2,000 kg. Das gesamte Pulvergemisch wird nochmals mittels eines Siebes 200 homogenisiert und anschließend in Hartgelantinekapseln zu 400 mg Nettogewicht eingefüllt und verschlossen. Das Überziehen mit einem magensaftresistenten Überzug erfolgt wie in Beispiel 2.

### Beispiel 4

### Herstellung von magensaftresistenten Mikrotabletten in Kapseln, enthaltend 87,0 Monoethylfumarat Ca-Salz, 120 mg Dimethylfumarat, 5,0 mg Monoethylfumarat Mg-Salz und 3,0 mg Monoethylfumarat Zn-Salz, entsprechend insgesamt 164 mg Fumarsäure ("Fortel"-Tabletten)

8,700 kg Monoethylfumarat Ca-Salz, 12,000 kg Dimethylfumarat, 0,500 kg Monoethylfumarat Mg-Salz, 0,30 kg Monoethylfumarat Zn-Salz werden zerkleinert, intensiv gemischt und mittels eines Siebs 800 unter entsprechenden Vorsichtsichtsmaßnahmen (Atemmaske, Handschuhe, Schutzanzug, etc.) homogenisiert. Es wird ein Hilfsstoffgemisch folgender Zusammensetzung hergestellt: 18,00 kg Stärkederivat (STA-RX 1500), 0,30 kg Cellulose mikrokristallin (Acivel PH 101), 0,75 kg PVP (Kollidon 120), 4,00 kg Primogel, 0,25 kg Kieselsäure kolloidal (Aerosil). Das gesamte Pulvergemisch wird mit dem Wirkstoffgemisch versetzt und mittels eines Siebes 200 homogenisiert und mit einer 2%-igen wäßrigen Lösung von Polyvinylpyrrolidon (Kollidon K25) auf übliche Weise zu einem Bindemittelgranulat verarbeitet und in trockenem Zustand mit der äußeren Phase gemischt. Diese besteht aus 0,50 kg Mg-Stearat und 1,50 kg Talkum. Das Pulvergemisch wird anschließend auf üblicher Weise zu gewölbten Mikrotabletten von 10,0 mg Bruttomasse und 2,0 mm Durchmesser gepreßt. Anstelle dieser klassischen Tablettiermethode können auch andere Methoden zur Herstellung von Tabletten verwendet werden, wie Direkttablettierung sowie feste Dispersionen nach der Schmelzmethode und die Sprühtrocknungsmethode.

Der magensaftresistente Überzug kann in einem klassischen Dragierkessel aufgeleert oder aufgesprüht sowie in einer Wirbelschichtapparatur aufgebracht werden. Zum Erreichen der Magensaftresistenz wird portionsweise eine Lösung von 2,250 kg Hydroxypropylmethylcellulosephthalat (HPMCP, Pharmacoat HP 50), in einem Gemisch folgender Lösungsmittel aufgelöst: Aceton 13,00 1, Ethanol 94 Gewichtsprozent denaturiert mit 2 % Keton 13,50 1 und Aqua demineralisata 2,50 1. Zu der fertigen Lösung wird als Weichmacher Rizinusöl 0,240 kg zugegeben und auf übliche Weise in Portionen auf die Tablettenkerne aufgetragen.

Filmcoat: Nach beendeter Trocknung wird anschließend in der gleichen Apparatur eine Suspension folgender Zusammensetzung als Filmcoat aufgetragen: Talk 0,340 kg, Titan (VI)-oxid Cronus RN 56 0,400 kg, Farblack L-Rotlack 86837 0,324 kg, Eudragit E 12,5 % 4,800 kg und Polyethlenglycol 6000 pH 11 XI 0,120 kg in einem Lösungsmittelgemisch folgender Zusammensetzung: 2-Propanol 8,170 kg, Aqua demineralisata 0,200 kg und Glycerintracetat (Triacetin) 0,600 kg.

Die magensaftresistenten Mikrotabletten werden anschließend in Hartgelantine-Steckkapseln zu 500,0 mg netto Gewicht eingefüllt und verschlossen.

### Beispiel 5

### Herstellung von Filmtabletten mit magensaftresistenten Überzug enthaltend 67,0 mg Monomethylfumarat-Ca-Salz, 30,0 mg Dimethylfumarat, 5,0 mg Monoethylfumarat-Mg-Salz und 3,0 mg Monoethylfumarat-Zn-Salz, entsprechend 75 mg Fumarsäure ("Mite"-Tabletten)

3,000 kg Dimethylfumarat, 6,700 kg Monoethylfumarat-Ca-Salz, 0,500 kg Monoethylfumarat-Mg-Salz und 0,300 kg Monoethylfumarat-Zn-Salz werden zerkleinert, intensiv gemischt und unter entsprechenden Vorsichtsmaßnahmen (Atemmaske, Handschuhe, Schutzanzug etc.) mittels eines Siebes 800 homogenisiert. Es wird ein Hilfsstoffgemisch folgender Zusammensetzung, auf ähnliche Weise wie unter Beispiel 4 aufgeführt, hergestellt, nämlich 30,000 kg Stärkederivat (STA-RX 1500®), 3,000 kg mikrokristalline Cellulose (Avicel PH 101®), 0,750 kg Polyvinylpyrrolidon (PVP, Kollidon®25), 4,000 kg Primogel, 0,250 kg kolloidale Kieselsäure (Aerosil®). Hilfsstoffe und Wirkstoffgemisch werden intensiv gemischt und mittels eines Siebes 200 homogenisiert. Das Ganze wird mit einer 2 %-igen wäßrigen Lösung von Polyvinylpyrrolidon (PVP, Kollidon®25) auf übliche Weise zu einem Bindemittelgranulat verarbeitet. Dem getrockneten Granulat wird eine Pulvermischung aus folgenden Hilfsstoffen als äußere Phase zugesetzt: 0,500 kg Mg-Stearat (Ph. Eur.) und 0,800 kg Talk (Ph. Eur. II).

Die homogene Granulatmischung wird zu gewölbten Tablettenkernen von 500,0 mg Gewicht und 11,5 mm Durchmesser auf übliche Weise komprimiert. Neben den Bindemittelmethoden können ebenfalls andere Tablettiermethoden, gemäß den Beispielen 1 und 4, Verwendung finden. Das Überziehen der Tablettenkerne mit einem magensaftresistenten Überzug sowie mit einem Filmcoat erfolgt sinngemäß wie unter den Beispielen 1 und 4 beschrieben.

Vorzugsweise werden die erfindungsgemäßen Zubereitungen peroral in Form von Tabletten oder Kapseln verabreicht, wobei diese festen Einzel-Arzneiformen vorzugsweise mit einem magensaftresistenten Überzug versehen wird, der sich nach der Magenpassage im Dünndarmsaft im Dünndarm innerhalb weniger Minuten löst und das aktive Prinzip aus der Arzneiform freisetzt. Zum systemischen Einstieg bzw. Ausstieg ist eine niedrige Dosierung (mite) erforderlich, für die therapeutische Dosierung nach der Einstiegsphase eine höhere Dosierung (forte).

### Beispiel 6

### Herstellung von Filmtabletten mit magensaftresistenten Überzug enthaltend 100,00 mg Monomethylfumarat-Ca-Salz, entsprechend 78 mg Fumarsäure

10,000 kg Monomethylfumarat-Calciumsalz werden zerkleinert, gemischt und mittels eines Siebes 800 unter entsprechenden Vorsichtsmaßnahmen (Atemmaske, Handschuhe, Schutzanzug etc.) homogenisiert. Anschließend wird ein Hilfsstoffgemisch folgender Zusammensetzung hergestellt: 21,000 kg Stärkederivat (STA-RX 1500®), 2,000 kg mikrokristalline Cellulose (Avicel PH 101®), 0,600 kg Polyvinylpyrrolidon (PVP, Kollidon®25), 4,000 kg Primogel, 0,300 kg kolloidale Kieselsäure (Aerosil®). Das gesamte Pulvergemisch wird mit dem Wirkstoff versetzt, gemischt und mittels eines Siebes 200 homogenisiert und mit einer 2 %-igen wäßrigen Lösung von Polyvidonpyrrohdon (Kollidon® K30) auf übliche Weise zu einem Bindemittelgranulat verarbeitet und in trockenem Zustand mit der äußeren Phase gemischt. Diese besteht aus 2,000 kg eines sogenannten FST-Komplexes, enthaltend 80 % Talk, 10 % Kieselsäure und 10 % Magnesiumstearat. Es wird anschließend auf übliche Weise zu gewölbten Tabletten von 400 mg Gewicht und 10 mm Durchmesser gepreßt. Anstelle dieser klassischen Tablettiermethoden können auch andere Methoden zur Herstellung von Tabletten angewendet werden, wie Direkttablettierung sowie feste Dispersionen nach der Schmelzmethode und der Sprühtrocknungsmethode. Das Überziehen der Tablettenkerne mit einem magensaftresistenten Überzug sowie mit einem Filmcoat erfolgt sinngemäß wie unter den Beispielen 1 und 4 beschrieben.

### Beispiel 7

### Herstellung von Filmtabletten mit magensaftresistentem Überzug enthaltend 50,0 mg Monomethylfumarat-Ca-Salz, 50,0 mg Dimethylfumarat 5,0 mg Monomethylfumarat-Mg-Salz und 3,0 mg Monomethylfumarat-Zn-Salz, entsprechend 85 mg Fumarsäure

5,000 kg Dimethylfumarat, 5,000 kg Monomethylfumarat-Ca-Salz, 0,500 kg Monomethylfumarat-Mg-Salz und 0,300 kg Monomethylfumarat-Zn-Salz werden zerkleinert, intensiv gemischt und unter entsprechenden Vorsichtsmaßnahmen (Atemmaske, Handschuhe, Schutzanzug etc.) mittels eines Siebes 800 homogenisiert. Es wird ein Hilfsstoffgemisch folgender Zusammensetzung auf ähnliche Weise wie in Beispiel 4 hergestellt, nämlich 19,000 kg Stärkederivat (STA-RX 1500®), 3,000 kg mikrokristalline Cellulose (Avicel PH 101®), 0,750 kg Polyvinylpyrrolidon (PVP, Kollidon®120), 4,000 kg Primogel, 0,250 kg kolloidale Kieselsäure (Aerosil®).

Hilfsstoffe und Wirkstoffgemisch werden intensiv gemischt und mittels eines Siebes 200 homogenisiert. Das Ganze wird mit einer 2 %-igen wäßrigen Lösung von Polyvinylpyrrolidon (PVP, Kollidon®25) auf übliche Weise zu einem Bindemittelgranulat verarbeitet und in getrocknetem Zustand mit der äußeren Phase gemischt. Diese besteht aus 0,500 kg Mg Stearat (Ph.Eur.) und 1,500 kg Talk (Ph.Eur. II).

Das ganze Granulat wird anschließend auf übliche Weise zu gewölbten Tabletten von 400 mg Gewicht und 10 mm Durchmesser gepreßt. Anstelle dieser klassischen Tablettiermethoden können auch andere Methoden zur Herstellung von Tabletten angewendet werden, wie Direkttablettierung sowie feste Dispersionen nach der Schmelzmethode und der Sprühtrocknungsmethode.

Das Überziehen der Tablettenkerne mit einem magensaftresistentem Überzug sowie mit einem Filmcoat erfolgt sinngemäß wie unter Beispielen 1 und 4 beschrieben.

### Beispiel 8

### Herstellung von Filmtabletten mit magensaftresistentem Überzug enthaltend 50,0 mg Mono-n-propylfumarat-Ca-Salz, entsprechend 32,8 mg Fumarsäure

5,000 kg Monopropylfumarat-Calciumsalz werden zerkleinert, gemischt und mittels eines Siebes 800 unter entsprechenden Vorsichtsmaßnahmen (Atemmaske, Handschuhe, Schutzanzug etc.) homogenisiert. Anschließend wird ein Hilfsstoffgemisch folgender Zusammensetzung hergestellt: 25,000 kg Stärkederivat (STA-RX 1500®), 3,000 kg mikrokristalline Cellulose (Avicel PH 101®), 0,600 kg Polyvinylpyrrolidon (PVP, Kollidon®25), 4,000 kg Primogel, 0,300 kg kolloidale Kieselsäure (Aerosil®). Das gesamte Pulvergemisch wird mit dem Wirkstoff versetzt, gemischt, mittels eines Siebes 200 homogenisiert und mit einer 2 %-igen wäßrigen Lösung von Polyvidonpyrrolidon (Kollidon ®K30) auf übliche Weise zu einem Bindemittelgranulat verarbeitet und in trockenem Zustand mit der äußeren Phase gemischt. Diese besteht aus 2,000 kg eines sogenannten FST-Komplexes, enthaltend 80 % Talk, 10 % Kieselsäure und 10 % Magnesiumstearat. Es wird anschließend auf übliche Weise zu gewölbten Tabletten von 400 mg Gewicht und 10 mm Durchmesser gepreßt. Anstelle dieser klassischen Tablettiermethoden können auch andere Methoden zur Herstellung von Tabletten angewendet werden, wie Direkttablettierung sowie feste Dispersionen nach der Schmelzmethode und der Sprühtrocknungsmethode. Das Überziehen der Tablettenkerne mit einem magensaftresistenten Überzug sowie mit einem Filmcoat erfolgt sinngemäß wie unter Beispielen 1 und 4 beschrieben.

### Beispiel 9

### Herstellung von magensaftresistenten Pellets in Kapseln enthaltend 50,0 mg Monomethylfumarat-Ca-Salz, 5,0 mg Monomethylfumarat-Mg-Salz und 3,0 mg Monomethylfumarat-Zn-Salz, entsprechend 45 mg Fumarsäure

5,000 kg Monomethylfumarat-Ca-Salz, 0,500 kg Monomethylfumarat-Mg-Salz und 0,300 kg Monomethylfumarat-Zn-Salz werden zerkleinert, intensiv gemischt und unter entsprechenden Vorsichtsmaßnahmen (Atemmaske, Handschuhe, Schutzanzug etc.) mittels eines Siebes 400 homogenisiert. Daneben werden 2 1 einer 20 % (m/V) Polyvinylpyrrolidon (Kollidon® K30) Lösung in Ethanol vorbereitet. 7,250 kg Nonpareilles Pellets werden in einem Dragierkessel belegt und mit einem Teil der Kollidon® K-30 Lösung besprüht bis diese leicht feucht werden. Das Wirkstoffgemisch wird danach portionsweise zugegeben bis zum Auftrocknen der Pellets. Diese Vorgehensweise der Befeuchtung/Auftrocknung wird bis zur endgültigen Zugabe des Wirkstoffgemisches weitergeführt. Der Rest der PVP Lösung wird mit 0,720 kg Eudragit E 12,5 % Lösung gemischt und ganz auf die Pellets gesprüht. Die Pellets werden letztlich bis zum vollständigen Austrocknen bewegt. Anstelle dieser Methode können auch andere weitere Methoden zur Pelletherstellung angewendet werden, wie das Wirbelschichtcoating, die Extrusion/ Spherozination-Methode. Weiter können auch die Pellets mit den einzelnen Wirkstoffen hergestellt und nach Befilmung (siehe unten) in den entsprechenden Verhältnissen zugemischt werden.

Die Pellets werden mit Eudragit S 12,5 % Lösung besprüht und mit Talk aufgetrocknet. Nach jedem Besprühungs-/Auftrocknungszyklus wird die Freisetzung des Wirkstoffs gemessen und Eudragit S 12,5 % Lösung/Talkum weiter zugegeben bis eine Freisetzung gemäß Spezifikation erhalten wird.

Die magensaftresistenten Pellets werden danach in Kapseln abgefüllt (146 mg Pellets/ Kapsel).

### Beispiel 10

### Herstellung von magensaftresitstenten Kapseln, enthaltend 50,0 mg Mono-isopropylfumarat-Ca-Salz, 50,0 mg Di-iso-propylfumarat, 5,0 mg Mono-iso-propylfumarat-Mg-Salz und 3,0 mg Mono-iso-propylfumarat-Zn-Salz, entsprechend 67 mg Fumarsäure

5,000 kg Mono-iso-propylfumarat-Ca-Salz, 5,000 kg Di-iso-propylfumarat, 0,500 kg Mono-iso-propylfumarat-Mg-Salz und 0,300 kg Mono-iso-propylfumarat-Zn-Salz werden zerkleinert, intensiv gemischt und unter entsprechenden Vorsichtsmaßnahmen (Atemmaske, Handschuhe, Schutzanzug etc.) mittels eines Siebes 800 homogenisiert. Diesem Wirkstoffgemisch wird ein homogenes Pulvergemisch folgender Zusammensetzung untergemischt: 32,200 kg sprühgetrocknete Lactose, 2,000 kg Cellulose, mikrokristallin (Avicel) und 1,000 kg kolloidale Kieselsäure (Aerosil), 1,000 kg Magnesiumstearat und 2,000 kg Talk. Das gesamte Pulvergemisch wird nochmals mittels eines Siebes 200 homogenisiert und anschließend in Hartgelantine-Steckkapseln zu 500 mg Nettogewicht eingefüllt und verschlossen.

Diese Kapseln können danach üblicherweise mit einem magensaftresistenten Überzug, bestehend aus Hydroxypropylmethylcellulosephthalat (HPMCP) und Rizinusöl als Weichmacher, versehen werden. Die Abfüllung kann ebenfalls anstelle von Hartgelatinekapseln in entsprechende magensaftresistente Kapseln, bestehend aus einem Gemisch von Celluloseacetatphthalat (CPA) und Hydroxypropylethylcelluloseacetatphthalat (HPMCP) erfolgen.

### Beispiel 11

### Herstellung von Mikropellets in Kapseln enthaltend 50,0 mg Methylhydrogenfumarat, entsprechend insgesamt 44,6 mg Fumarsäure

5,000 kg Methylhydrogenfumarat werden zerkleinert und mittels eines Siebes 400 unter entsprechenden Vorsichtsmaßnahmen (Atemmaske, Handschuhe, Schutzanzug etc.) homogenisiert. Daneben werden 2 1 einer 20 %-igen (m/V) Polyvinylpyrrolidon (Kollidon® K30) Lösung in Ethanol vorbereitet. 7,250 kg Nonpareilles Pellets werden in einem Dragierkessel belegt und mit einem Teil der Kollidon K30-Lösung besprüht bis diese leicht feucht werden. Das Wirkstoffgemisch wird danach portionsweise zugegeben bis zum Auftrocknen der Pellets. Diese Vorgehensweise der Befeuchtung/Auftrocknung wird bis zur endgültigen Zugabe des Wirkstoffgemisches weitergeführt. Die Pellets werden letztlich bis zum vollständigen Austrocknen bewegt. Anstelle dieser Methode können auch andere weitere Methoden zur Pelletherstellung angewendet werden, wie das Wirbelschichtcoating, die Extrusion/Spherozination-Methode. Weiter können auch die Pellets mit entsprechenden Verhältnissen zugemischt werden.

Die Pellets werden danach in Kapseln abgefüllt (126,5 mg Pellets/Kapsel).

### Anwendungsbeispiele

Am Modell der Nierentransplantation bei Ratten wurde die Wirkung von Calciummethylfumarat zur Linderung der Host-versus-Graft-Reaktion sowohl im akuten als auch im chronischen Rejektionsmodell untersucht.

### Akutes Rejektionsmodell

Zur Untersuchung des Einflusses von Calciummethylfumarat auf die akute Abstoßung des Transplantats wurden Tierversuche (Ratte) durchgerührt. Hierzu wurden zwei Gruppen von Ratten über einen Zeitraum insgesamt von 56 Tagen (28 Tage vor der Nierentransplantation (-28) bis 28 Tage nach Transplantation (+28)) mit Calciummethylfumarat (CaMF, Dosis: 33,3 mg/kg/Tag) oder Placebo behandelt. In der Placebo-Gruppe waren n = 9, in der Verum-Gruppe n = 12 auswertbare Tiere. Anschließend wurde die Überlebenszeit der Tiere in Tagen nach der Transplantation ausgewertet. Die Ergebnisse der Versuche sind in Tabelle 1 dargestellt.

Aus der signifikanten Erhöhung der mittleren Überlebenszeit in Tagen bei Verabreichung von Calciummethylfumarat ist ersichtlich, daß dieses die Abstoßung unterdrücken kann, also die Host-versus-Graft-Reaktion im für die Transplantationsmedizin positiven Sinne beeinflußt.

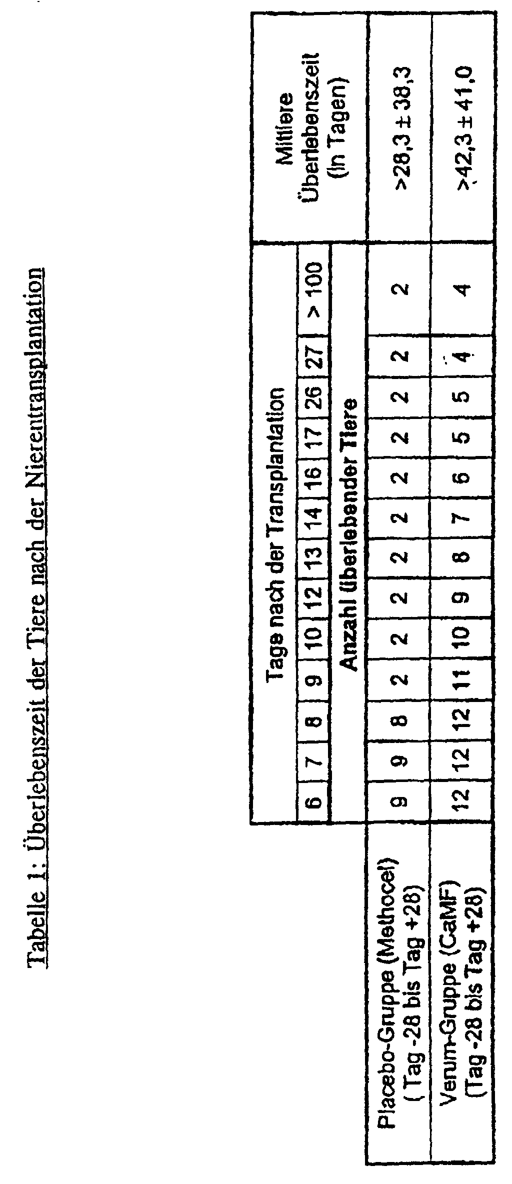

### Chronisches Rejektionsmodell

Zur Untersuchung der Wirkung von Calciummethyfumarat auf die chronische Abstoßung wurden im Tierversuch 3 Gruppen von Ratten transplantiert (Niere). Dabei wurde in den Gruppen 1 und 2 jeweils vom Tag 28 vor der Transplantation bis Tag 28 nach der Transplantation Placebo (Gruppe 1) bzw. Calciummethylfumarat (CaMF) als Wirkstoff (Gruppe 2) verabreicht. Der dritten Gruppe wurde CaMF an den Tagen 30 bis 60 nach der Nierentransplantation verabreicht. Alle Tiere erhielten Cyclosporin an den Tagen 0 bis 9 nach der Transplantation. Die verabreichten Dosen betrugen 1,5 mg/kg/Tag Cyclosporin (subkutan) und 33,3 mg/kg/Tag CaMF (peroral).

Die Kontrolle der Behandlung erfolgte durch Messung der Kreatininwerte im Serum zunächst an den Tagen 0, 1, 3, 5 und 10, dann wöchentlich sowie der Kontrolle der Proteinwerte im Urin, die wöchentlich gemessen wurden. In der Gruppe 1 und 2 waren jeweils n = 10 Tiere und in der Gruppe 3 waren n = 9 Tiere auswertbar. Die Ergebnisse der Behandlung in der 10. Woche nach Transplantation sind in Tabelle 2 gegeben.

Nach bisher 10 Wochen follow-up zeigen die Placebo-behandelten Tiere signifikant erhöhte Serumkreatininwerte und eine vermehrte Proteinurie gegenüber den beiden Verum-Gruppen, wobei die vorbehandelte Gruppe 2 noch bessere Werte als die später behandelte Gruppe 3 zeigt. Die vorliegenden Ergebnisse deuten auf eine signifikante Hemmung der Nierenschädigung durch Calciummethylfumarat hin.

**Tabelle 2:**

| chronisches Rejektionsmodell | | |
|---|---|---|
| | Kreatin (µmol/l) | Proteinurie (mg/24 h) |
| Gruppe 1: Methocel (Tag -28 bis Tag +28 | 92,2±60,8 | 76,1±40,6 |
| Gruppe 2: CaMF Tag -28 bis Tag +28 | 55,7±9,6 | 31,8±18,4 |
| Gruppe 3: CaMF (Tag +30 bis Tag +60) | 88,0±42,4 | 52,5±31,6 |

## Patentansprüche

1. Verwendung eines oder mehrerer Fumarsäure-C₁₋₅-monoalkylester(s) in Form eines Salzes mit ein- oder zweiwertigen Kationen oder in Form der freien Säure, allein oder in Kombination mit einem Di-C₁₋₅-alkylfumarar zur Herstellung eines Arzneimittels zur Behandlung der Transplantatabstoßung (Host-versus-Graft-Reaktionen) bei Organ- und Zelltransplantationen.

2. Verwendung eines oder mehrerer Salze ein- oder zweiwertiger Kanonen von Fumarsäuremonoalkylestern der allgemeinen Formel 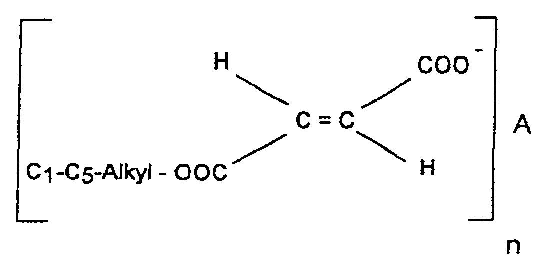 gegebenenfalls in Gemisch mit Dialkylfumarat der Formel 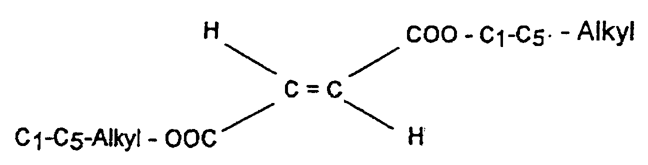 wobei A ein zweiwertiges Kation der Reihe Ca, Mg, Zn oder Fe bzw, ein einwertiges Kation aus der Reihe Li, Na oder K und n die Zahl 1 oder 2 je nach Art des Kations bedeuten,
und/oder eines oder mehrerer Alkylhydrogenfumarate der allgemeinen Formel 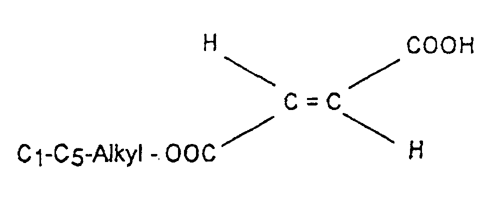 gegebenenfalls im Gemisch mit Dialkylfumarat der Formel 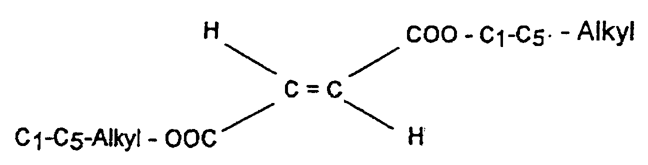 und gegebenenfalls üblichen pharmazeutischen Hilfs- und Trägerstoffen zur Herstellung eines Arzneimittels zur Behandlung von Host-versus-Graft-Reaktionen gemäß Anspruch 1.

3. Verwendung nach Anspruch 1 oder 2, **dadurch gekennzeichnet, daß** es sich um das Calciumsalz des Fumärsäuremonomethyl- oder -ethylesters handelt.

4. Verwendung nach Anspruch 1 oder 2, **dadurch gekennzeichnet, daß** es sich um eines oder mehrere der Calcium-, Magnesium- und/oder Zinksalze des Fumarsäuremonoethylesters im Gemisch mit Dimethylfumarat handelt.

5. Verwendung nach einem der Sprüche 1 bis 4, bei der die Wirkstoffe oral in Form von Tabletten, von Mikrotabletten, Pellets oder Granulaten in Kapseln oder Kapseln verabreicht werden.

6. Verwendung nach Anspruch 5, **dadurch gekennzeichnet, daß** es sich um das Calciumsalz des Fumarsäuremonoalkylesters in einer Menge von 10 mg bis 300 mg handelt, wobei das Gesamtgewicht der Wirkstoffe 10 bis 300 mg beträgt.

7. Verwendung nach Anspruch 5, **dadurch gekennzeichnet, daß** es sich um 10 bis 290 Gewichtsteile des Calciumsalzes des Fumarsäuremonoalkylescers und 290 bis 10 Gewichtsteile Dimethylfumarat handelt, wobei das Gesamtgewicht der Wirkstoffe 20 bis 300 mg beträgt.

8. Verwendung nach Anspruch 5, **dadurch gekennzeichnet, daß** es sich um 10 bis 250 Gewichtsteile des Calciumsalzes des Fumarsäuremonoalkylesters, 1 bis 50 Gewichtsteile Dimethylfumarat und 1 bis 50 Gewichtsteile des Zinksalzes des Fumarsäuremonoalkylesters handelt, wobei das Gesamtgewicht der Wirkstoffe 20 bis 300 mg beträgt.

9. Verwendung nach Anspruch 5, **dadurch gekennzeichnet, daß** es sich um 10 bis 250 Gewichtsteile des Calciumsalzes des Fumarsäuremonoalkylesters, 250 bis 10 Gewichtsteile Dimethylfumarat, 1 bis 50 Gewichtsteile des Magnesiumsalzes des Fumarsäuremonoalkyesters und 1 bis 50 Gewichtsteile des Zinksalzes des Fumarsäuremonoalkylesters handelt, wobei das Gesamtgewicht der Wirkstoffe 30 bis 300 mg beträgt.

10. Verwendung nach einem der Ansprüche 1 bis 9, **dadurch gekennzeichnet, daß** die Behandlung in Kombination mit einem Immunsuppresivum erfolgt.

11. Verwendung nach Anspruch 10, **dadurch gekennzeichnet, daß** eine sequenzielle bzw, alternierende Applikation von Cyclosporin mit der Applikation der Fumarsäureverbindungen erfolgt.

12. Verwendung nach einem der Ansprüche 1 bis 9, **dadurch gekennzeichnet, daß** die Behandlung in Kombination mit einem oder mehreren Präparaten der Transplantationsmedizin erfolgt.

13. Verwendung nach einem der Ansprüche 5 bis 12, **dadurch gekennzeichnet, daß** die Größe bzw. der mittlere Durchmesser von Pellecs oder Mikrotabletten im Bereich von 300 bis 2000 µm, insbesondere im Bereich von 500 µm bis 1500 µm oder 1000 µm liegt.

14. Verwendung nach Anspruch 5, **dadurch gekennzeichnet, daß** die Arzneimittel in Form von Weich- oder Harrgelanrinekapseln eingesetzt werden.

15. Verwendung nach den Ansprüchen 1 bis 14, **dadurch gekennzeichnet, daß** die Dosiseinheiten der Arzneimittel mit einem magensaftresistenten Überzug versehen sind.

16. Verwendung nach einem der Ansprüche 1 bis 4, **dadurch gekennzeichnet, daß** die Arzneimittel in Form von Präparaten für die kutane und transdermale Verabreichung, Präparaten für die parencerale Verabreichung und Präparaten für die rektale Verabreichung eingesetzt werden.

## Claims

1. The use of one or more fumaric acid-C₁-C₅-monoalkylester(s) in the form of a salt with mono- or bivalent cations or in the form of free acid, either alone or in combination with a di-C₁-C₅-alkylfumarate for treating host-versus-graft rcactions in organ and cell transplantation.

2. The use of one or more salts of mono- or bivalent cations of fumaric acid monoalkyl esters of the general formula 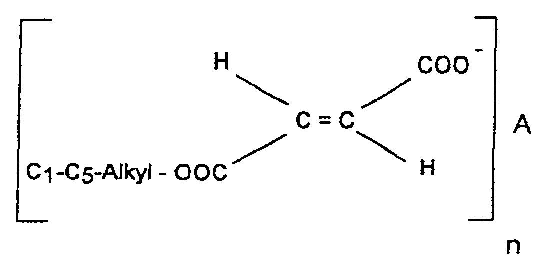 optionally in admixture with dialkyl fumarate of the formula 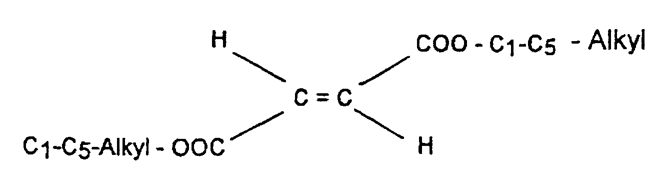 wherein A is a bivalent cation from the series consisting of Ca, Mg, Zn or Fe or a monovalent cation from the series Li, Na or K, respectively, and n denotes the numeral 1 or 2 depending on the type of cation and/or one or more alkyl hydrogen fumarates of the general formula 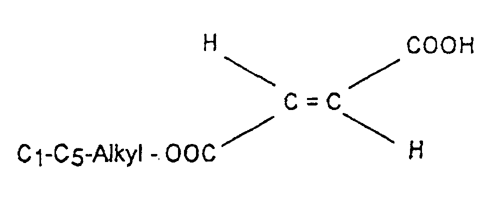 optionally in admixture with dialkyl fumarate of the formula 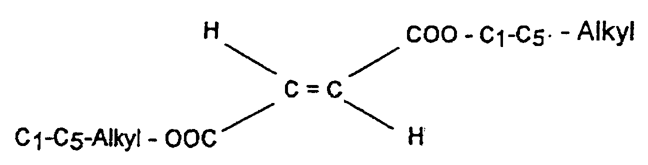 and, optionally, commonly used pharmaceutical excipients and vehicles for manufacture of a medicament for the treatment of host-versus-graft reactions according to claim 1.

3. The use according to claim 1 or 2, **characterised in that** the calcium salt of fumaric acid monomethyl or ethyl ester is used.

4. The use according to claim 1 or 2, **characterised in that** one or more of the calcium, magnesium and/or zinc salts of fumaric acid monoethyl ester in admixture with dimethyl fumarate is used.

5. The use according to any of the claims 1 to 4, **characterised in that** the active ingredients are administered orally in the form of tablets, micro-tablets, pellets or granulates in capsules or capsules.

6. The use according to claim 5, **characterised in that** the calcium salt of the fumaric acid monoalkyl ester is used in an amount of 10 to 300 mg, the total weight of the active ingredients being 10 to 300 mg.

7. The use according to claim 5, **characterised in that** 10 to 290 parts by weight of the calcium salt of the fumaric acid monoalkyl ester and 290 to 10 parts by weight of dimethyl fumarate are used, the total weight of the active ingredients being 20 to 300 mg.

8. The use according to claim 5, **characterised in that** 10 to 250 parts by weight of the calcium salt of the fumaric acid monoalkyl ester, 1 to 50 parts by weight dimethyl fumarate and 1 to 50 parts by weight of the zinc salt of the fumaric acid monoalkyl ester are used, the total weight of the active ingredients being 20 to 300 mg.

9. The use according to claim 5, **characterised in that** 10 to 250 parts by weight of the calcium salt of the fumaric acid monoalkyl ester, 250 to 10 parts by weight dimethyl fumarate, 1 to 50 parts by weight of the magnesium salt of the fumaric acid monoalkyl ester and 1 to 50 parts by weight of the zinc salt of the fumaric acid monoalkyl ester are used, the total weight of the active ingredients being 30 to 300 mg.

10. The use according to any of the claims 1 to 9, **characterised in that** treatment is carried out in combination with an immunosuppressive agent.

11. The use according to claim 10, **characterised in that** a cyclosporine is applied in sequential or alternating manner with the application of the fumaric acid compounds.

12. The use according to any of the claims 1 to 9, **characterised in that** the treatment is carried out in combination with one or more preparations used in transplantation medicine.

13. The use according to any of the claims 5 to 12, **characterised in that** the size or the mean diameter, respectively, of pellets or micro-tablets is in the range of 300 to 2000 µm, especially in the range of 500 to 1,500 µm or 1,000 µm.

14. The use according to claim 5, **characterised in that** the drugs are employed in the form of soft or hard gelatine capsules.

15. The use according to any of the claims 1 to 14, **characterised in that** the dosage units of the drugs are provided with an enteric coating.

16. The use according to any of the claims 1 to 4, **characterised in that** the drugs are employed in the form of preparations for cutaneous and transdermal administration, preparations for parenteral administration and preparations for rectal administration.

## Revendications

1. Utilisation d'un ou de plusieurs fumarates de monoalkyle en C₁ à C₅ sous la forme d'un sel avec des cations mono ou divalents ou sous la forme de l'acide libre, seuls ou en combinaison avec un fumarate de dialkyle en C₁ à C₅ pour la préparation d'un médicament pour le traitement du rejet de greffe (réactions de l'hôte contre le greffon) dans les transplantations d'organes et de cellules.

2. Utilisation d'un ou de plusieurs sels de cations mono ou divalents de fumarates de monoalkyle de formule générale 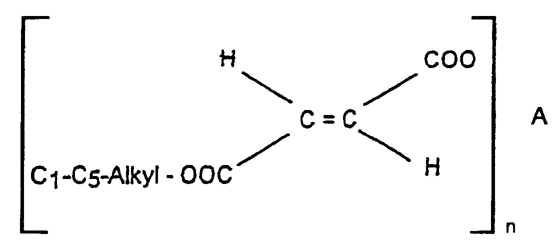 éventuellement en mélange avec du fumarate de dialkyle de formule 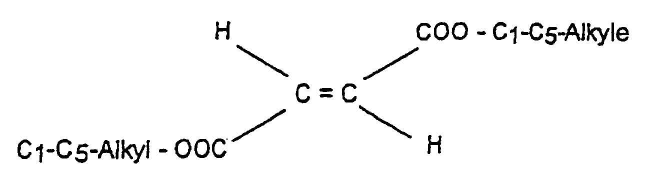 dans laquelle A représente un cation divalent de la série Ca, Mg, Zn ou Fe ou un cation monovalent de la série Li, Na ou K et n représente le nombre 1 ou 2 selon respectivement le type du cation,
et/ou un ou plusieurs hémifumarate d'alkyle de formule générale 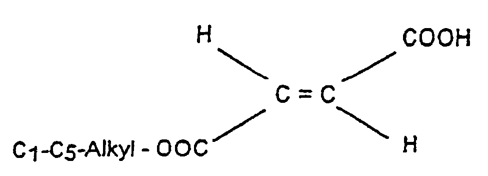 éventuellement en mélange avec du fumarate de dialkyle de formule 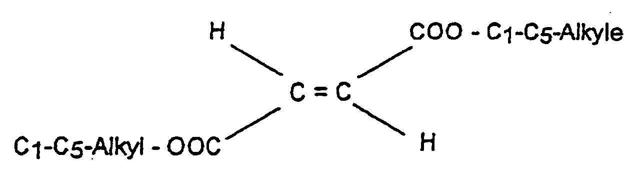 et éventuellement des adjuvants et supports pharmaceutiques usuels pour la préparation d'un médicament pour le traitement de réactions de l'hôte contre le greffon selon la revendication 1.

3. Utilisation selon la revendication 1 ou 2, **caractérisée en ce qu'**il s'agit du sel de calcium de l'hémifumarate de méthyle ou d'éthyle.

4. Utilisation selon la revendication 1 ou 2, **caractérisée en ce qu'**il s'agit d'un ou de plusieurs des sels de calcium, de magnésium et/ou de zinc de l'hémifumarate d'éthyle en mélange avec le fumarate de diméthyle.

5. Utilisation selon une des revendications 1 à 4 dans laquelle on administre les substances actives par voie orale sous forme de comprimés, de microcomprimés, de pastilles ou de granulés en capsules ou de capsules.

6. Utilisation selon la revendication 5, **caractérisée en ce qu'**il s'agit du sel de calcium du fumarate de monoalkyle en une quantité de 10 mg à 300 mg, dans laquelle la masse totale des substances actives est de 10 à 300 mg.

7. Utilisation selon la revendication 5, **caractérisée en ce qu'**il s'agit de 10 à 290 parties en poids du sel de calcium du fumarate de monoalkyle et de 290 à 10 parties en poids de fumarate de diméthyle, dans laquelle la masse totale des substances actives est de 20 à 300 mg.

8. Utilisation selon la revendication 5, **caractérisée en ce qu'**il s'agit de 10 à 250 parties en poids du sel de calcium du fumarate de monoalkyle, de 1 à 50 parties en poids de fumarate de diméthyle et de 1 à 50 parties en poids du sel de zinc du fumarate de monoalkyle, dans laquelle la masse totale des substances actives est de 20 à 300 mg.

9. Utilisation selon la revendication 5, **caractérisée en ce qu'**il s'agit de 10 à 250 parties en poids du sel de calcium du fumarate de monoalkyle, de 250 à 10 parties en poids de fumarate de diméthyle, de 1 à 50 parties en poids du sel de magnésium du fumarate de monoalkyle et de 1 à 50 parties en poids du sel de zinc du fumarate de monoalkyle, dans laquelle la masse totale des substances actives est de 30 à 300 mg.

10. Utilisation selon une des revendications 1 à 9, **caractérisée en ce qu'**on administre le traitement en combinaison avec un immunosuppresseur.

11. Utilisation selon la revendication 10, **caractérisée en ce qu'**on effectue un administration séquentielle ou alternée de cyclosporine avec l'administration des composés d'acide fumarique.

12. Utilisation selon une des revendications 1 à 9, **caractérisée en ce qu'**on administre le traitement en combinaison avec une ou plusieurs préparations de la médecine des transplantations.

13. Utilisation selon une des revendications 5 à 12, **caractérisée en ce que** la taille ou le diamètre moyen des pastilles ou des microcomprimés se situe dans la gamme de 300 à 2000 µm, en particulier dans la gamme de 500 µm à 1 500 µm ou 1 000 µm.

14. Utilisation selon la revendication 5, **caractérisée en ce qu'**on utilise le médicament sous forme de capsules de gélatine molle ou dure.

15. Utilisation selon les revendications 1 à 14, **caractérisée en ce que** les unités de dose des médicaments sont munies d'un revêtement résistant au suc gastrique.

16. Utilisation selon les revendications 1 à 4, **caractérisée en ce qu'**on utilise les médicaments sous la forme de préparations pour l'administration cutanée et transdermique, de préparations pour l'administration parentérale et de préparations pour l'administration rectale.
